# EUROPEAN PATENT APPLICATION

(11) **EP 4 435 106 A1**
(43) Date of publication of application: **25.09.2024**
(21) Application number: 22893306.5
(22) Date of filing: 14.11.2022
(51) Int. Cl.: C12N 15/115, A61K 31/7088, A61K 31/375, A61P 25/28, A23L 33/13

(54) **NOVEL APTAMER, AND COMPOSITION FOR COGNITIVE FUNCTION IMPROVEMENT AND ANTI-AGING COMPRISING APTAMER AS ACTIVE INGREDIENT**

(30) Priority: 15.11.2021 KR 20210156501
(71) Applicant: Nexmos Co., Ltd., Gyeonggi-do 16827 (KR)
(72) Inventor: KIM, Ji Hyun, Suwon-si Gyeonggi-do 16380 (KR); CHOI, Sol Lp, Seongnam-si Gyeonggi-do 13186 (KR); KIM, Naegauridoemeulwihayeomideumi, Yongin-si Gyeonggi-do 16892 (KR)
(74) Representative: FRKelly
(86) International application number: PCT/KR2022/017898
(87) International publication number: WO 2023/085887

(57) **Abstract**

The present invention relates to an aptamer set forth in SEQ ID NO: 1, and a composition for improving cognitive function and anti-aging comprising vitamin C the aptamer as active ingredients, wherein the aptamer of the present invention has a superior oxidative delay effect on vitamin C compared to other known aptamers, and the aptamer and/or vitamin C complex has improved cognitive function and anti-aging effects in aging animal models, and thus may be used in pharmaceutical, food, and/or cosmetic fields.

## Description

### [Technical Field]

The present invention relates to a novel aptamer and a composition for improving cognitive function and anti-aging that includes the aptamer as an active ingredient.

### [Background Art]

According to data from the South Korean National Statistical Office, 13.8% of the population aged 65 or older in Korea has already entered a rapidly aging society in 2017, and it is estimated that 47.7% of households aged 65 or older will be aged by 2045, if aging continues as it is now. The Aging World 2015 report released by the U.S. National Statistical Office in 2016 also predicted that Korea's population aged 65 or older will be 35.9% (second only to Japan among 141 countries surveyed) by 2050, and Korea's aging rate is the fastest in the world.

As aging progresses, cognitive function, including information processing and learning, perception, reasoning, problem solving, and memory, naturally decreases, and cognitive decline is more pronounced than other age groups in old age when aging progresses rapidly. The decline in physical function in the elderly acts in combination with aging phenomena and negatively affects brain function, and the decline in brain function causes serious cognitive decline, making it impossible to maintain normal daily life. The decline in senile cognition eventually leads to the isolation of individuals from social activities and severe damage to the quality of life in old age.

Among cognitive functions, memory and learning are known to be formed in the hippocampal formation consisting of hippocampus, dentate gyrus, and subiculum. When a specific nerve cell located in the hippocampal formation is repeatedly stimulated with a high-frequency current, LTP (long-term potentiation) phenomenon that EPSP (excitatory post-synaptic potential) increases for a long time in adjacent nerve cells connected to synapses has been reported. This LTP phenomenon means that the ability to communicate with neighboring nerve cells connected by synapses is strengthened and it is accepted as a physiological mechanism of memory/learning and oblivion along with LTD (long-term depression), the opposite phenomenon of LTP.

The decrease in memory and learning ability is one of the functional changes in the brain caused by aging, and it is known that the synaptic density between hippocampal neurons rapidly decreases as aging progresses. Accordingly, synaptic plasticity ability also significantly decreases, and as aging progresses, greater stimulation is required to induce LTP, or it is easier to fall into LTD and this decrease in LTP has been reported as a major cause of senile cognitive decline.

The rapid aging and the increase in life expectancy of the elderly, along with the growing social interest in the quality of life, are amplifying the demand to live a long and healthy life, and accordingly, there is an urgent need to develop health foods and drugs that can improve and prevent cognitive decline in the elderly. Currently, several drugs and health foods have been released to improve cognitive decline in the elderly, but they are concentrated in certain senile diseases such as Alzheimer's, so the continuous search for active substances that can effectively improve cognitive decline caused by natural aging is required.

On the other hand, aptamers are nucleic acid molecules that have a specific binding affinity for molecules through interactions other than typical Watson-Crick base pairing.

The aptamer may specifically bind to a selected target and may regulate the activity of the target, for example, through binding, it may block the target's ability to function. The aptamer, which is generated from a pool of irregular sequence oligonucleotides by an in vitro selection process, is generated over one hundred proteins including growth factors, transcription factors, enzymes, immunoglobulins, and receptors. A typical aptamer is 10-15 kDa (30-45 nucleotides) in size, binds to the target with an affinity of sub-nanomolar, and is distinguished from closely related targets (e.g., aptamer typically does not bind to other proteins from the same gene family). A series of structural studies have shown that aptamers can use the same types of binding interactions that induce affinity (e.g., hydrogen bonds, electrostatic complementarity, hydrophobic contacts, steric exclusion) and specificity in antibody-antigen complexes.

Aptamer has a number of desirable properties for use as therapeutic and diagnostic agents, including high specificity and affinity, biological efficacy, and excellent pharmacokinetic properties. In addition, aptamer offers specific competitive advantages over antibody and other protein biology. For example, it is as follows. 1)Speed and control.

The aptamers are created entirely by an in vitro process, allowing the rapid generation of initial leads, including therapeutic leads. In vitro selection allows tight control of the aptamer's specificity and affinity, and allows the production of leads, including leads to both toxic and non-immunogenic targets. 2)Toxicity and immunogenicity.

Aptamer as a class exhibits little or no toxicity or immunogenicity. When aptamer high doses (90 days of 10 mg/kg daily) were administered to rats and woodchucks for a long period of time, no toxicity was observed in either clinical, cellular, or biochemical measurements. It is dramatically difficult to induce antibodies to aptamer because the efficacy of many monoclonal antibodies is severely limited by the immune response of the antibody itself, while reliably aptamer cannot be produced by T-cells via MHC, and the immune response is trained not to recognize nucleic acid fragments. 3) administration.

Most currently authorized antibody treatments are administered intravenously (usually over 2-4 hours), while aptamer can be administered by subcutaneous injection (Aptamer bioavailability via subcutaneous administration in monkey study is >80%).

This difference is originally due to the relatively low solubility and thus the need for large volumes for most therapeutic MAbs. With good solubility (>150 mg/mL) and relatively low molecular weight (Aptamer: 10-50 kDa; Antibody: 150 kDa), aptamer may be transported by weekly administration by injection in a volume of 0.5 mL or less. In addition, small sized aptamer allows penetration into a conformational-limiting region, but antibody or antibody fragment cannot penetrate, which still provides other advantages of aptamer-based treatment or prevention. 4) Scalability and cost.

Therapeutic aptamers are chemically synthesized and thus may be scaled as easily as necessary to meet production requirements. Difficulties in scaling production currently limit the usefulness of some biology and the capital cost of mass-scale protein production plants is enormous, while a single mass-scale oligonucleotide synthesizer can be produced by raising 100 kg/year and requires an initial investment that is low. Continuous improvement in process development is expected to lower the cost of resources to <$100/g. 5) Stability.

Therapeutic aptamers are chemically solid. They are essentially adapted to restore activity after exposure to factors such as heat or denaturing agents and can be stored at room temperature for an extended period (>1 yr) as lyophilized powder.

### [Prior art]

Korea Patent Publication No. 1020180056190

### [Disclosure]

### [Technical Problem]

The object of the present invention is to provide an aptamer having cognitive function improvement and anti-aging effects.

Another object of the present invention is to provide a novel composition for improving cognitive function.

Another object of the present invention is to provide a novel anti-aging composition.

Another object of the present invention is to provide a composition for treating and/or improving dementia.

### [Technical Solution]

To achieve the above object, the present invention provides an aptamer comprising the nucleotide sequence set forth in SEQ ID NO: 1 that delays the oxidation of vitamin C.

In addition, the present invention provides a pharmaceutical composition for improving cognitive function comprising vitamin C and the aptamer of the present invention as active ingredients.

In addition, the present invention provides a pharmaceutical composition for anti-aging, comprising vitamin C and the aptamer of the present invention as active ingredients.

In addition, the present invention provides a food composition for improving cognitive function comprising vitamin C and the aptamer of the present invention as active ingredients.

In addition, the present invention provides an anti-aging food composition comprising vitamin C and the aptamer of the present invention as active ingredients.

In addition, the present invention provides a pharmaceutical composition for treating dementia comprising vitamin C and the aptamer of the present invention as active ingredients.

In addition, the present invention provides a dementia-relieving food composition comprising vitamin C and the aptamer of the present invention as an active ingredient.

Hereinafter, the present invention will be described.

### Pharmaceutical composition using Aptamer and/or vitamin C complex

When the composition of the present invention is a pharmaceutical composition, it may be used for the desired indication of the present invention. For the administration of the composition of the present invention, in addition to the above-described active ingredients, pharmaceutically acceptable carriers, excipients, or diluents may be included. The carriers, excipients, and diluents may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methylhydroxybenzoate, propylhydroxybenzoate, talc, magnesium stearate, and mineral oil.

The pharmaceutical composition of the present invention may be used after being formulated in the form of topical formulation, suppository formulation, a sterile injection solution or an oral formulation such as powder, a granule agent, a tablet, a capsule, a suspension, an emulsion, a syrup, an aerosol, respectively, according to a conventional method. Specifically, the formulation may be prepared by using a diluent or an excipient such as a filler, a weighting agent, a binder, a wetting agent, a disintegrating agent, a surfactant, or the like. Solid formulations for oral administration include tablets, pills, powders, granules, capsules, etc., but are not limited thereto. Such solid formulations may be prepared by mixing at least one excipient, for example, starch, calcium carbonate, sucrose, lactose, gelatin, etc., in addition to the above active ingredients. Also, lubricants such as magnesium stearate and talc may also be used in addition to simple excipients. In addition, various excipients, such as wetting agents, sweeteners, fragrances, preservatives, etc., may be added to prepare the preparations in addition to the liquid preparations for oral administration and liquid paraffin. Formulations for parenteral administration include sterilized aqueous solutions, non-aqueous solvents, suspensions, emulsions, and lyophilized preparations. As the non-aqueous solvent and the suspension, vegetable oils such as propylene glycol, polyethylene glycol, and olive oil, injectable esters such as ethylolate, and the like may be used. As the suppository base, witepsol, macrosol, Twin 61, cacao oil, laurin oil, glycerogelatin, and the like may be used.

The appropriate dose of the pharmaceutical composition of the present invention varies depending on the patient's condition and body weight, the degree of disease, the drug type, and the time, but may be appropriately selected by those skilled in the art. The daily dose of the composition is preferably 0.001 mg/kg body weight to 500 mg/kg body weight and may be divided into once to several times a day as needed.

### Food and beverage and food composition using aptamer and/or vitamin C complex

The present invention provides a food composition or a beverage composition comprising the aptamer of the present invention alone or the aptamer and the vitamin C complex as an active ingredient.

In an embodiment of the present invention, the composition preferably further includes at least one of collagen, elastin, hyaluronic acid, and peptides, but is not limited thereto.

In one embodiment of the present invention, the food composition is preferably snacks, candies, dairy products, gum products, pastes, breads, or ice cream, but is not limited thereto.

### Application of Aptamer-Based Cosmetics

The present invention provides a cosmetic composition comprising an aptamer that reduces the oxidation rate of vitamin C and an additional ingredient.

The additional ingredient of the present invention may use any raw material used in cosmetics regardless of any kind of extract or active material. For example, green tea extract, licorice extract, mulberry extract, white mulberry extract, Scutellaria radix extract, pueraria extract, red ginseng extract that is good for whitening; apricot extract, orange extract, lemon extract, guava extract, rosemary extract, cornelian extract, Ganoderma extract, ginkgo extract, seoshiokyoksan extract, consonant extract that is good for preventing aging; quince, Opuntia ficus-indica var saboten extract, paprika extract, aloe extract, scrubber extract, seaweed extract extract that is good for moisturizing; carrot extract, soybean extract, grapefruit seed extract, grape seed extract, Portulaca oleracea extract with antioxidant effect; Caviar, pomegranate, ginseng extract to help improve wrinkles; peach extract Cnidium Rhizome extract to help regenerate skin; Centella extract, chamomile extract, Lithospermum erythrorhizon root extract, sophora extract, angelica extract that good for atopy; mint extract, Saururus Herba extract, houttuynia extract, paeony extract that good for acne; liquid smoke, dandelion extract, karendula extract, Amur cork tree skin extract, trifoliate orange extract, fennel extract, compuri extract with anti-inflammatory and antibacterial activity; chestnut skin extract and green tea extract with pore tightening activity; glycerin, panthenol, hyaluronic acid, ceramide, beta glucan that helps moisturize; arbutin, vitamin C, whitense, retinol, astaxanthin, resveratrol, polyphenol with whitening activity; elastin, collagen, coenzyme Q10, effectin, EGF that good for elasticity; anti-inflammatory and antibacterial such as propolis, allantoin, phytostan, infraacid; antioxidant vitamin E (natural tocopherol), rosemary oil extract, and grapefruit seed extract are applied.

Suitable cosmetic formulations of the present invention may be provided in the form of, for example, cream, skin, gel, semi-solid or solid form, skin, lotion, powder, ointment, spray, or comseal stick. In addition, it may be prepared in the form of foam or an aerosol composition further comprising a compressed propellant.

In addition, the cosmetic composition of the present invention may further comprises fatty substances, solubilizers, concentrators and gelling agents, softeners, antioxidants, suspensions, stabilizers, foaming agents, fragrances, surfactants, water ionic or nonionic emulsifiers, fillers, metal ion blockers and chelating agents, preservatives, vitamins, blockers, wetting agents, essential oils, dyes, pigments, hydrophilic or lipophilic activators, lipid follicles, or adjuvants commonly used in cosmetic or dermatological fields. In addition, the above components may be introduced in an amount commonly used in the field of dermatological science.

The cosmetic composition of the present invention may be prepared in any formulation conventionally manufactured in the art, and may include formulations such as skin lotion, skin softener, skin toner, astringent, lotion, milk lotion, moister lotion, nutritional lotion, massage cream, nutritional cream, moister cream, hand cream, essence, nutritional essence, pack, soap, shampoo, cleansing foam, cleansing cream, body lotion, body cleanser, bath aid, etc.

### [Advantageous Effects]

According to the present invention, the aptamer (GCCAGTCTCGCGGTGGCGGC) of the specific sequence of the present invention has a superior oxidative delay effect on vitamin C compared to other known aptamers, and because the aptamer and/or vitamin C complex with the aptamer of the present invention has improved cognitive function and anti-aging effects in an aging animal model, it can be used in various formulations of functional cosmetics, various health drinks, antioxidant drinks, and antioxidant foods by maintaining the reduction state of vitamin C and maintaining its antioxidant function for a long time. It can also be applied as a candidate substance that requires cognitive improvement and anti-aging effects.

### [Description of Drawings]

Figure 1 is about aptamin C332 (left) and aptamin C320 (right), and when analyzing the structure of 32mer with mFold, the secondary structure of two stem loop structures was confirmed, and 20mer (aptamin C320) maintaining this stem loop was designed.
Figure 2 shows the confirmation of vitamin C oxidation delay effect of aptamin C320 through OPDA assay,

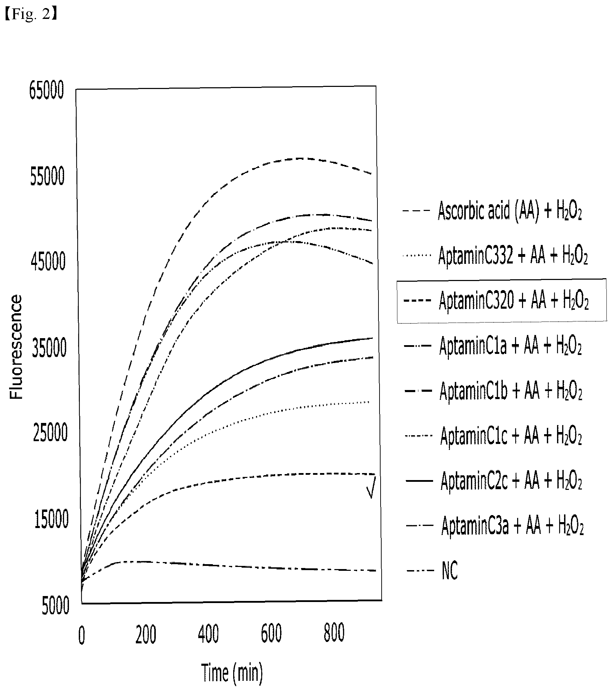

   In Fig. 2, each sequence is as follows, and in the 20mer DNA from C320 to C3a below, the portion where the sequence overlaps with the 32mer DNA(C332) is underlined,
      C332, GTGGAGGCGGTGGCCAGTCTCGCGGTGGCGGC (SEQ ID NO: 2)
      C320, GCCAGTCTCGCGGTGGCGGC (SEQ ID NO: 1)
      C1a, GAGGCGGTGGCCAGTCTCAT (SEQ ID NO: 3)
      C1b, GCCGCGGCGGTGGCCGCGGC (SEQ ID NO: 4)
      C1c, GGAGGCGGTGGCCAGTCTCA (SEQ ID NO: 5)
      C2c, GAGCTCGCGCCGGAGTTCTC (SEQ ID NO: 6)
      C3a, GGCGGTGGCCAGTCTCGCGG (SEQ ID NO: 7)
   In the graph of FIG. 2, the part marked with '√ ' is the C320 aptamer + AA+H₂O₂ treatment group.

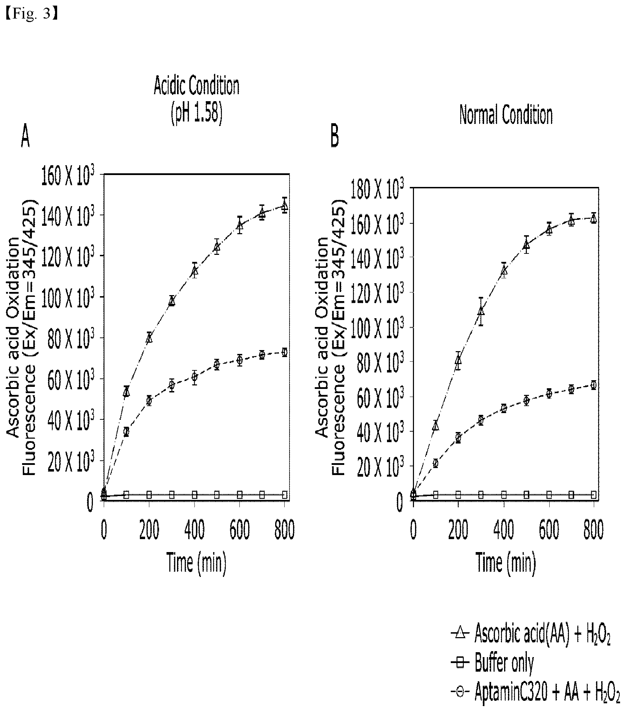

Figure 3 shows the maintenance of vitamin C oxidation delay effect of aptamin C320 in a gastric acid-like environment through OPDA assay.

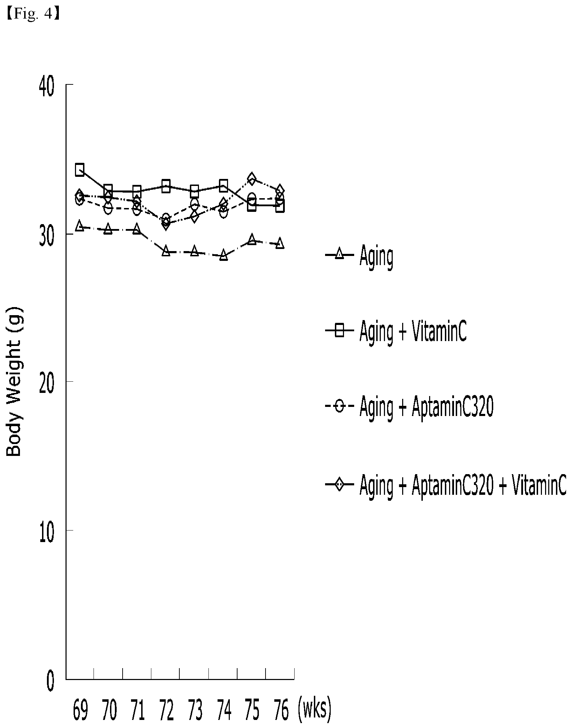

Figure 4 shows the changes in the weight of the aged animals during the administration period.

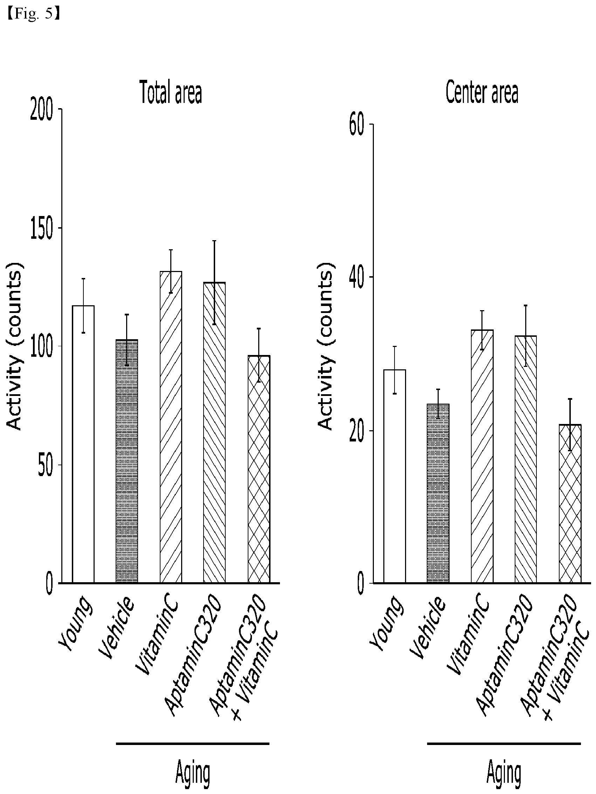

Figure 5 shows the measurement of general mobility through the Open field test.

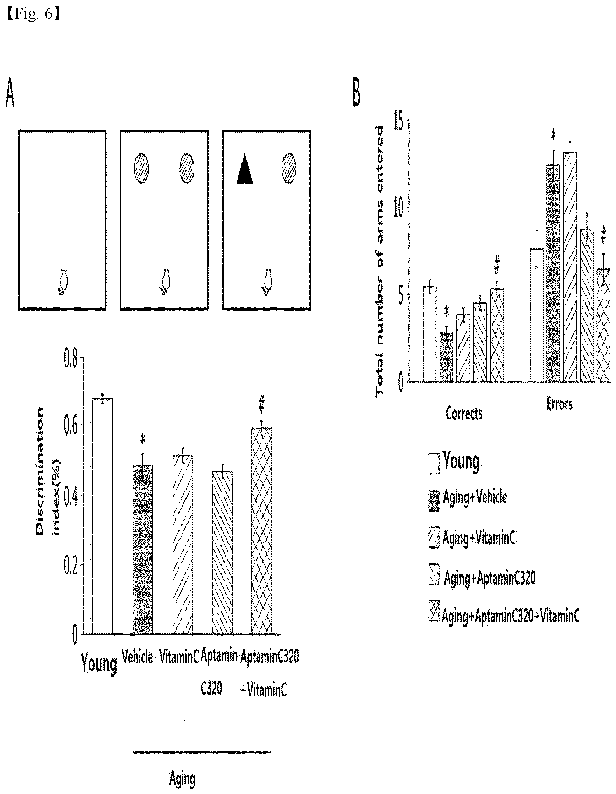

Figure 6 shows the evaluation of cognitive memory and learning ability through the Novel object recognition test (A) and the radial 8-arm measure test (B).

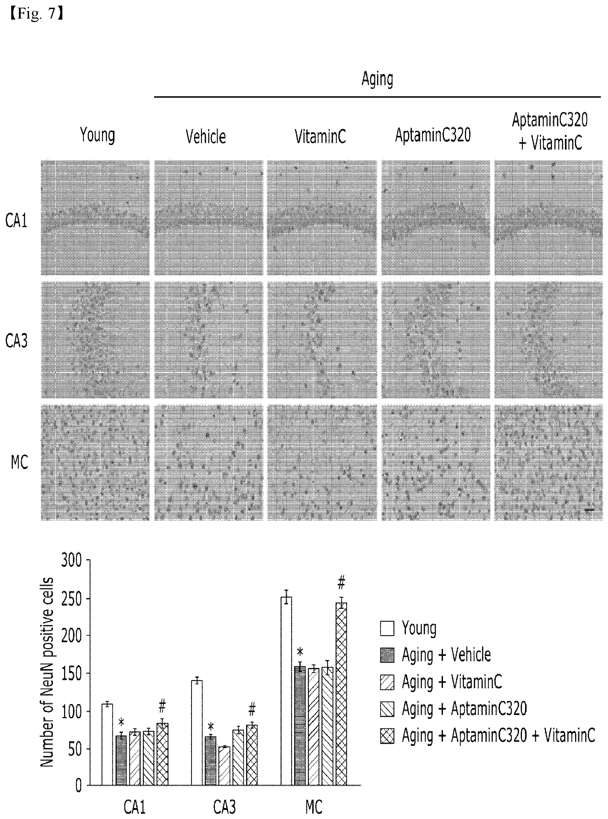

Figure 7 shows the observation of changes in nerve cells in hippocampus (CA1, CA3) and motor cortex (MC) in the brain using NeuN staining.

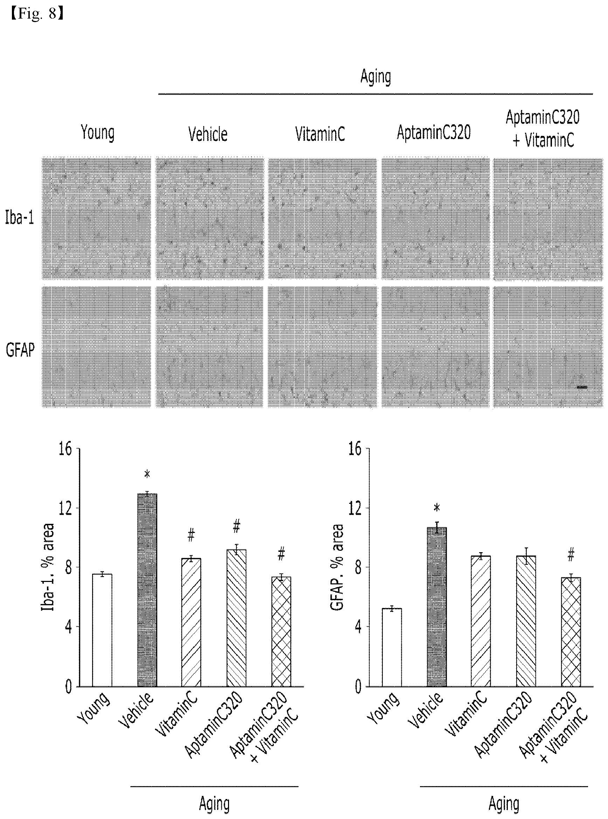

Figure 8 shows the observation of changes in microglia in hippocampus in the brain using Iba-1, GFAP staining.

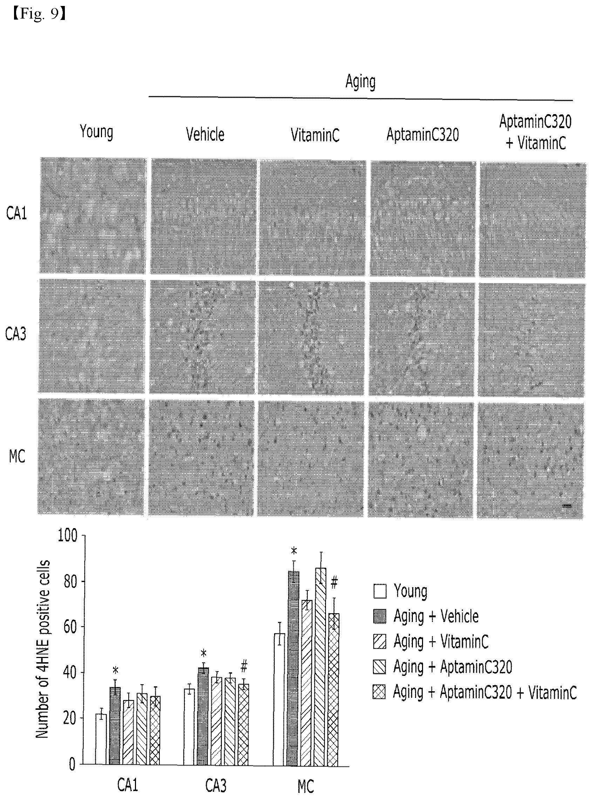

Figure 9 shows the observation of oxidative stress changes in hippocampus and mortar cortex areas in the brain with 4HNE staining.

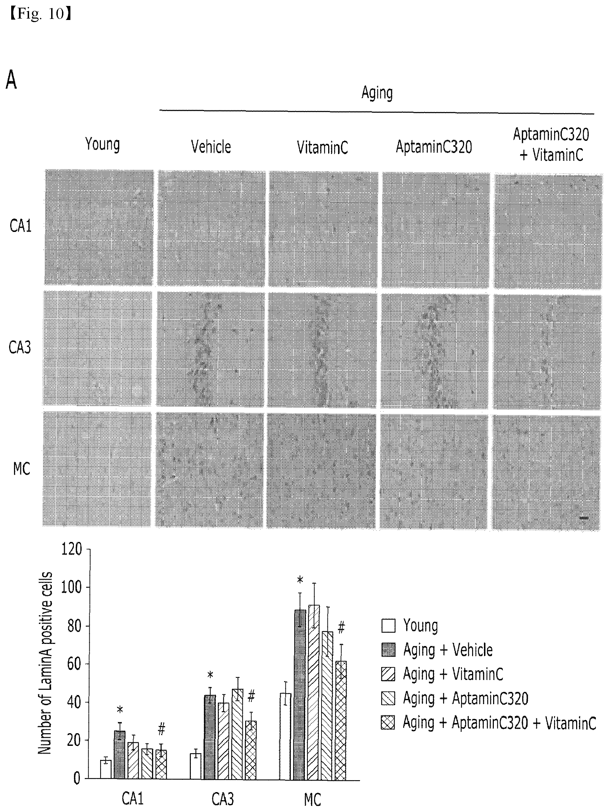

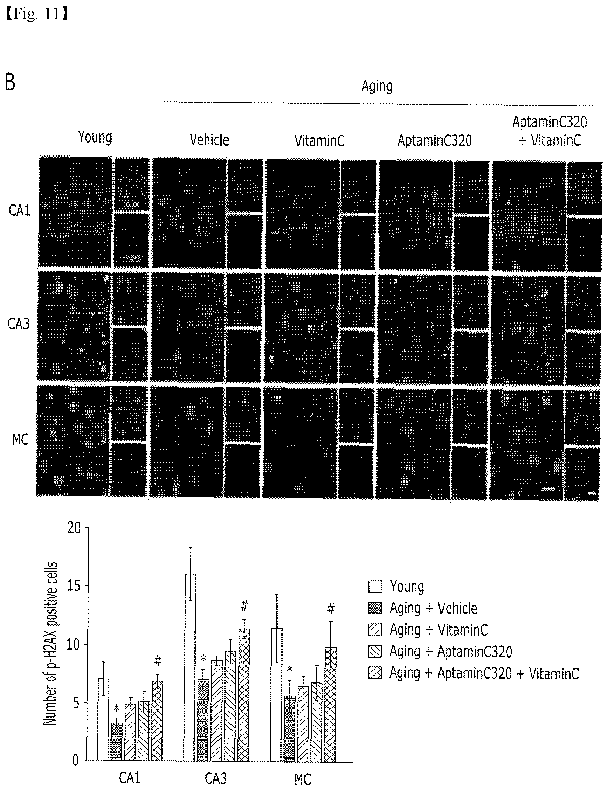

Figures 10 and 11 show changes in DNA damage in hippocampus and motor cortex in the brain through Lamin A (FIG. 10) and p-H2AX (FIG. 11) staining.

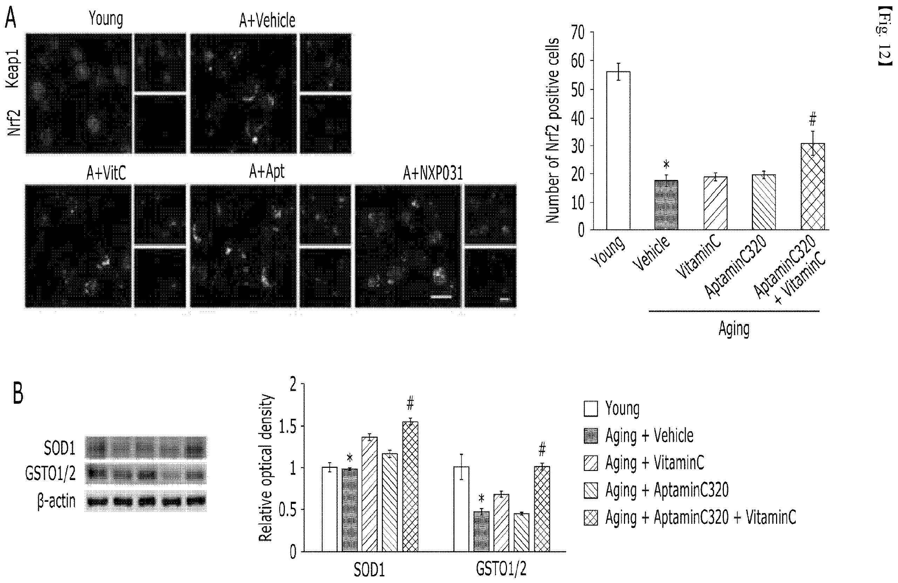

Figure 12 shows changes in antioxidant activity in vivo through confirmation of Nrf2/keap1, SOD1, and GSTO1/2.

### [Mode for Invention]

Hereinafter, the present invention will be described in more detail through examples, but these are merely illustrative and are not intended to limit the scope of the present invention. It is obvious to those skilled in the art that the embodiments described below can be modified without departing from the essential gist of the invention.

### Example 1. Screening of Materials

The aptamer was discovered through SELEX. However, aptamer C320 is made by cutting the length of aptamer that binds to vitamin C of 32mer (C332) possessed by Nexmos into 20mer. For comparative experiments, 20mer aptamers were synthesized and used.

### 1) Structural Analysis

Sequence-based secondary structure analysis was performed using the mFold program. Through analysis of the secondary structure of the 32mer DNA aptamer, it was cut into 20mer DNA maintaining the secondary structure.

### 2)OPDA assay

After heating the aptamer dissolved in annealing buffer to 95°C, the temperature is gradually lowered to room temperature to form a secondary structure of the aptamer, and then mixed with the reduced L-ascorbic acid to react for about 30 minutes so that the aptamer can bind to the L-ascorbic acid. In this case, the L-ascorbic acid was mixed at a ratio of 0.1% and the aptamer was mixed at 0.001%. Thereafter, hydrogen peroxide was added to prepare an oxidation condition, and then oxidation of L-ascorbic acid was measured by adding o-phenylenediamine (OPDA), a fluorescent dye. The degree of DHA production may be quantitatively analyzed by measuring the amount of fluorescence from DHA-OPDA generated by the reaction of DHA, an oxide of L-ascorbic acid, with OPDA. Under the above conditions, the amount of fluorescence of DHA-OPDA was measured every 2 minutes for a total of 900 minutes.

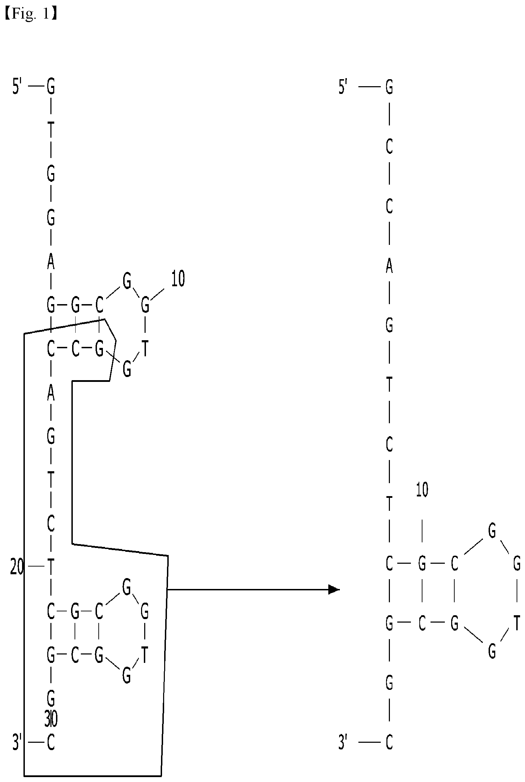

The results are shown in FIGS. 1 and 2.

Figure 1 is about aptamin C332 (left) and aptamin C320 (right), and when analyzing the structure of 32mer with mFold, the secondary structure of two stem loop structures was confirmed, and 20mer (aptamin C320) maintaining this stem loop was designed.

Figure 2 shows the confirmation of vitamin C oxidation delay effect of aptamin C320 through OPDA assay,

In Fig. 2, each sequence is as follows, and in the 20mer DNA from C320 to C3a below, the portion where the sequence overlaps with the 32mer DNA(C332) is underlined,
C332, GTGGAGGCGGTGGCCAGTCTCGCGGTGGCGGC (SEQ ID NO:
2)
C320, GCCAGTCTCGCGGTGGCGGC (SEQ ID NO: 1)
C1a, GAGGCGGTGGCCAGTCTCAT (SEQ ID NO: 3)
C1b, GCCGCGGCGGTGGCCGCGGC (SEQ ID NO: 4)
C1c, GGAGGCGGTGGCCAGTCTCA (SEQ ID NO: 5)
C2c, GAGCTCGCGCCGGAGTTCTC (SEQ ID NO: 6)
C3a, GGCGGTGGCCAGTCTCGCGG (SEQ ID NO: 7)

As shown in FIG. 2, it is confirmed through OPDA assay whether the existing 32mer (Aptamin C332) and aptamer C320 having a length shortened to 20mer may delay the oxidation of vitamin C, and other aptamers in which aptamer C is cut to 20mer are tested together as a control group. As shown in FIG. 2, aptamer C320 delays the oxidation of vitamin C to the highest extent.

Therefore, among several 20mer aptamers, aptamin C320 was used as a candidate material and subsequent experiments were conducted.

### Example 2, Confirmation of maintenance of effect upon oral ingestion

OPDA assay was used to confirm whether the vitamin C oxidation delay effect of aptamin C320 was maintained in a low pH environment similar to gastric acid in order to confirm that the effect of the substance was not reduced or eliminated by the pH environment in the stomach when ingested orally.

Briefly, the aptamer dissolved in annealing buffer was heated to 95°C and then slowly lowered the temperature to room temperature to form a secondary structure of the aptamer, and then mixed with reduced L-ascorbic acid to react for about 30 minutes so that the aptamer could be combined with L-ascorbic acid. Thereafter, hydrogen peroxide was added to prepare an oxidation condition, and at this time, the pH was adjusted to be between 1.4 and 1.6 using hydrochloric acid, and oxidation of L-ascorbic acid was measured by adding an o-phenylenediamine (OPDA), which is a fluorescent dye. The degree of DHA production can be quantitatively analyzed by measuring the amount of fluorescence from DHA-OPDA generated by the reaction of DHA, an oxide of L-ascorbic acid, with OPDA. Under the above conditions, the fluorescence amount of DHA-OPDA was measured every 2 minutes for a total of 900 minutes.

The results are shown in Fig. 3.

FIG. 3 is a diagram showing the maintenance of vitamin C oxidation delay effect of aptamin C320 in a gastric acid-like environment through OPDA assay, and as shown in FIG. 3, it is confirmed that the vitamin C oxidation delay effect of aptamin C320 is maintained without significantly decreasing in acid condition compared to normal condition. Therefore, since the effect of aptamin C320 is maintained even under strong acidic conditions, it is expected that the intake of aptamin C320 will not lose its effectiveness as it passes through the gastrointestinal tract.

### Example 3. Confirmation of effectiveness in the aging experimental animal model

For the behavior test, open field test, novel object recognition test, and radial 8-arm maze test were performed,

Immuno-histochemstry experiments using NeuN markers were conducted in relation to changes in nerve cells in hippocampus (CA1, CA3) and motor cortex (MC) in the brain,

Immuno-histochemstry experiments using Iba-1, and GFAP markers were conducted to determine changes in microglia in the brain in hippocampus,

Observation of oxidative stress changes in hippocampus and mortar cortex areas in the brain was conducted by Immuno-histochemstry experiment using 4HNE markers,

Identification of changes in DNA damage in hippocampus and motor cortex in the brain was conducted with Immunohistochemistry using Lamin A as a marker, and Immunofluorescence using phospho-histone 2AX (p-H2AX) markers,

Immunofluorescence and western blot were performed using Nrf2, Keap1, SOD1, and GSTO1/2 as antioxidant markers to confirm the change in antioxidant capacity in vivo.
In summary,

### 1)Animals

To apply the specific physical conditions in old age as a standard, imported animals from CRJ, Japan, were introduced. Male B6J, 70 weeks old, was imported through Orient Bio. After customs clearance, imported animals will be delivered to this laboratory using a vibration-free electric car, and two animals per cage were separated, and raised (23±2°C, 50±1%, 12-hour light/dark cycles) in a constant temperature and humidity device maintained in which the set environment was maintained. Water and feed were consumed freely.

### 2) Experiment

After 1 week of adaptation to the experimental environment, 10 B6J mice are randomly assigned. Afterwards, all groups of mice were administered orally every day for 8 weeks with the aptamer C320/vitamin C complex, aptamer C320 alone, and vitamin C alone according to the intervention of each group. After applying the experimental intervention for 2 weeks, cognitive function is evaluated through behavioral experiments. In the behavioral experiment, the cognitive function of the Novel object recognition test and 8 arm radial maze test was confirmed, and the exercise function was confirmed by performing an open field test. The day after the behavioral experiment was completed, inhalation anesthesia was performed using ethyl ether, and cervical dislocation was performed without pain to obtain tissue.

### 3) Histological analysis

To confirm histological differences, the animals were sacrificed and then the tissues were collected. Brain tissues were obtained and fixed in 10% formalin solution for 1 hour. The tissues immobilized on formalin were paraffin blocks and cut into 5 µm thicknesses from the outside to prepare a section. Paraffin was removed with xylene, washed with alcohol and distilled water, and then the markers of nerve (NeuN), inflammation (Iba-1, GFAP), oxidation (4-HNE), antioxidant (Nrf2/Keap1), and aging (Lamin A, p-H2AX) were stained with primary antibodies. After the secondary antibody reaction, data were collected by observing under a microscope.

### 4) Western blotting

Brain tissue was put in lysis buffer (Trizma base, NaCl, 10% NP40, 10% Na-deoxycholate, 100 mM EDTA, and 10% SDS) containing a proteolysis inhibitor and ground to prepare a homogenate. Then, it was put in a centrifuge and separated at a speed of 13,000 rpm for 30 minutes while maintaining 4°C. The supernatant was taken to obtain protein, and the protein was separated on SDS PAGE gel in a uniform amount of protein by electrophoresis. Thereafter, the protein was transferred from the gel to the PVDF membrane, blocked with 5% skim milk for 1 hour, and reacted with the primary antibody overnight at 4°C. The primary antibodies reacted with SOD-1 and GSTO1/2 to confirm the antioxidant effect. Each reacted with the secondary antibody to which HRP was bound at room temperature for 2 hours, and then reacted with an enhanced chemiluminescence (ECL) solution to be developed on a film. The amount of expressed target protein is compared with a ratio to β-actin, which is a positive control group, and the amount of expression is measured using the Image J program.

### 5) Statistical Analysis

To evaluate the therapeutic effects of aptamin C320 and vitamin C complex, analysis was performed using the SPSS 25.0 program. One way ANOVA was performed if the collected data were normality, and Kruskal wallis test was performed if not. When differences between groups occurred, Tukey test was performed as a post-test. The significance of statistical differences was limited to less than p-value 0.05. All data values are expressed as mean values ± standard errors (S.E.M.). (*p<0.05, completed with the Young group. #p<0.05, completed with the Aging group)

In this example, the drug dose is
200 mg/kg of vitamin C,
Aptamin C320 4 mg/kg,
Aptamin C320+Vitamin C 4mg/200mg/kg,

The results of the above example will be described below.

FIG. 4 is a graph showing a change in the weight of the aged animal during the administration period, and the weight of the aged animal is measured once a week during the administration period of 8 weeks. As shown in FIG. 4, the weight change of the animal due to the substance administration is not shown, and there is no significant difference between groups.

FIG. 5 is a graph showing measurement of general mobility through an Open field test, and general and spontaneous mobility is analyzed through an Open field test. As shown in FIG. 5, there is no statistically significant difference between the entire groups in the pattern in which the animal moves the entire space and the central portion of the space. Therefore, it is considered that there is no significant difference in spontaneous mobility.

Figure 6 shows the evaluation of cognitive memory and learning ability through the Novel object recognition test and the radial 8-arm maze test,

### [A] Novel object recognition test.

The Novel object recognition test is a cognitive memory evaluation model using the characteristics of mice curious about new things. As shown in FIG. 6A, the aging-vehicle group showed a decrease in cognitive function compared to the young group, and the aging-AptaminC320+VitaminC group showed more interest in new substances and improved cognitive memory compared to the aging-vehicle group.

### [B] Radial 8-arm maze test

The radial 8-arm maze test is a method of measuring spatial learning ability, and re-entering a previously visited arm in a maze of eight arms is calculated as an error and is calculated as the number of correct choices until the first error occurs. As may be seen from FIG. 6B, in a radial 8-arm maze, the aging-vehicle group made fewer correct choices compared to the young group, and a larger number of errors occurred. The aging-AptaminC320+VitaminC group made a larger number of correct choices and a smaller number of error choices in comparison.

Therefore, it is believed that spatial learning and memory functions have been improved by ingestion of the AptaminC320+VitaminC complex.

FIG. 7 shows the observation of changes in nerve cells in hippocampus (CA1, CA3) and motor cortex (MC) in the brain by NeuN staining, and CA1, CA3, and motor cortex regions of the hippocampus participate in space and working memory and are important for memory search and formation. As shown in FIG. 7, NeuN is a marker of nerve cells, and NeuN-positive cells are significantly reduced in the aging group, but the number of NeuN-positive cells is increased in the group taking AptaminC320+VitaminC. Therefore, it is confirmed that intake of the AptaminC320+VitaminC complex suppresses nerve cell death due to aging.

FIG. 8 shows the observation of changes in microglia in hippocampus in the brain using Iba-1 and GFAP staining, and microglia and astroglia increase when damage to the central nervous system and stress increase. As shown in FIG. 8, Iba-1, an inflammatory marker of microglia, and GFAP, an inflammatory marker of astrocytes, are increased with aging and statistically significantly reduced with the intake of AptaminC320+Vitamin C complex.

FIG. 9 shows observation of changes in oxidative stress in hippocampus and motor cortex regions in the brain through 4HNE staining, and 4HNE is an oxidative stress biomarker produced by lipid peroxidation. As shown in FIG. 9, it was confirmed that 4HNE-positive cells were increased in the hippocampus CA1, CA3, and motor cortex regions, indicating that oxidative stress was increased in senescent cells. Ingestion of the AptaminC320+VitaminC complex resulted in a statistically significant decrease of 4HNE accumulated in senescent cells.

Figures 10 and 11 show the confirmation of DNA damage changes in hippocampus and motor cortex in the brain through Lamin A (FIG. 10) and p-H2AX (FIG. 11) staining,

[FIG. 10] Lamin A is an index indicating aging-related nuclear damage and accumulates during natural aging and is activated when cells are exposed to oxidative stress. It was confirmed that the expression of Lamin A was increased in Hippocampus CA1, CA3, and motor cortex of aging animals compared to that of young animals. As shown in FIG. 10, Lamin A-positive cells were statistically significantly decreased in the AptaminC320+Vitamin C complex group compared to the aging group.

[FIG. 11] p-H2AX is closely related to repair of damaged DNA. Aged mice showed statistically significantly reduced expression of p-H2AX in hippocampus CA1, CA3, and motor cortex regions compared to younger mice. However, the expression of p-H2AX was statistically significantly increased by taking AptinC320+VitaminC complex.

FIG. 12 shows changes in antioxidant activity in vivo through confirmation of Nrf2/keap1, SOD1, and GSTO1/2 expression, and Nrf2 is a transcription factor that regulates the expression of various antioxidant genes and is associated with cell defense and regulation of antioxidant mechanisms.

As shown in FIG. 12, in the aging group, Nrf2 is mainly located in the cytoplasm, and its expression is decreased as compared with that in the younger group. In contrast, taking an AptaminC320+VitaminC complex increases the expression of Nrf2 reduced by aging. In addition, it is confirmed that SOD-1 and GSTO1/2, which are antioxidants regulated by Nrf2, are increased as compared with the aging group.

## Claims

1. An Aptamer consisting of the sequence set forth in SEQ ID NO: 1 that delays the oxidation of vitamin C.

2. A pharmaceutical composition for improving cognitive function comprising vitamin C and the aptamer of claim 1 as active ingredients.

3. A pharmaceutical composition for anti-aging, comprising vitamin C and the aptamer of claim 1 as active ingredients.

4. A food composition for improving cognitive function comprising vitamin C and the aptamer of claim 1 as active ingredients.

5. An anti-aging food composition comprising vitamin C and the aptamer of claim 1 as active ingredients.

6. A pharmaceutical composition for treating dementia comprising vitamin C and the aptamer of claim 1 as active ingredients.

7. A dementia-relieving food composition comprising vitamin C and the aptamer of the claim 1 as active ingredients.
